# EUROPEAN PATENT SPECIFICATION

(11) **EP 3 023 706 B1**
(45) Date of publication and mention of the grant of the patent: **03.01.2018**
(21) Application number: 15194480.8
(22) Date of filing: 13.11.2015
(51) Int. Cl.: F24F 11/00

(54) **METHOD, APPARATUS, DEVICE AND COMPUTER PROGRAM FOR ACQUIRING AIR QUALITY**
VERFAHREN, VORRICHTUNG, GERÄT UND COMPUTERPROGRAMM ZUR ERFASSUNG DER LUFTQUALITÄT
PROCÉDÉ, APPAREIL, DISPOSITIF ET PROGRAMME D'ORDINATEUR POUR LE RELEVÉ DE LA QUALITÉ DE L'AIR

(30) Priority: 21.11.2014 CN 201410675672
(43) Date of publication of application: 25.05.2016
(73) Proprietor: Xiaomi Inc., Beijing 100085 (CN)
(72) Inventor: ZHANG, Bin, 10085 BEIJING (CN); SU, Jun, 10085 BEIJING (CN); XIA, Yongfeng, 100085 BEIJING (CN); HU, Yunlin, 10085 BEIJING (CN); WANG, Yang, 10085 BEIJING (CN); CHEN, Hao, 10085 BEIJING (CN)
(74) Representative: Studio Torta S.p.A.

(56) References cited:
- CN-A- 103 685 655
- CN-A- 103 760 859
- CN-U- 203 101 358
- US-A1- 2002 081 964

## Description

### TECHNICAL FIELD

The present invention generally relates to a field of monitoring technology, and more particularly to a method, an apparatus, a device and a computer program for acquiring air quality.

### BACKGROUND

With the constant improvement of industrialization degree, air pollution is more and more serious. However, in order to improve air quality, an air purifier has been popularized in each family.

The typical air purifier is usually provided with an air quality detection device, which may detect air quality of the surrounding environment.

In the process of implementing the present disclosure, it is found that at least the following problems exist in the related art: when the air is not circulated, dust particles may float in the air and is almost at a stationary state, and thus the air quality detection device may not accurately measure the content of the dust particles, whereby the measured air quality in general will be superior to the actual air quality, that is, the accuracy of the measured air quality is lower. Patent document CN 103760859 discloses an exhaust monitoring system for outdoor use. However, the above issues remain unsolved.

### SUMMARY

In order to solve the problem in the related art that the accuracy of the measured air quality is lower, the present disclosure provides a method, an apparatus, a device and a computer program for acquiring air quality as defined in claims 1, 6, 11 ans 12. The technical solutions are put forward as follows.

According to a first aspect of the present invention, there is provided a method for acquiring air quality, which is applied in a household appliance including a fan and an air quality detection device, the method including:
controlling the fan to rotate;
detecting air quality by the air quality detection device; and
generating air quality information according to the detection result of the air quality detection device.

The detecting the air quality by the air quality detection device includes:
detecting whether the rotation time of the fan reaches a predetermined length of time; and
detecting the air quality by the air quality detection device if the rotation time reaches the predetermined length of time.
The controlling the fan to rotate includes:
detecting whether an airflow speed in a current environment reaches a predetermined speed; and
controlling the fan to rotate if the airflow speed does not reach the predetermined speed.

Optionally, the method further includes:
receiving a trigger signal sent by a mobile terminal for triggering the detection of the air quality; and
performing an operation of controlling the fan to rotate after receiving the trigger signal.

Optionally, the method further includes:
directly sending the air quality information to the mobile terminal which is used for displaying the air quality information;
   and/or,
sending the air quality information to a server which is used for forwarding the air quality information to the mobile terminal for displaying the air quality information;
   and/or,
sending the air quality information to a home gateway device which is used for forwarding the air quality information to the mobile terminal which is used for displaying the air quality information.

Optionally, the method further includes:
detecting whether a predetermined time is reached, the predetermined time being a time determined at a predetermined interval, or, the predetermined time being a detection time set by a user; and
performing an operation of controlling the fan to rotate if the predetermined time is reached.

Optionally, the method further includes:
sending the air quality information to a server, which is used for forwarding all of the air quality information received before or the air quality information received last time to a mobile terminal after receiving a trigger signal sent by the mobile terminal for displaying the received air quality information;
   and/or,
sending the air quality information to a home gateway device, which is used for forwarding all of the air quality information received before or the air quality information received last time to a mobile terminal after receiving a trigger signal sent by the mobile terminal for displaying the received air quality information.

According to a second aspect of the present invention, there is provided an apparatus for acquiring air quality, for a household appliance including a fan and an air quality detection device, the apparatus comprising:
a rotation control module configured to control the fan to rotate;
an air quality detection module configured to detect air quality by the air quality detection device; and
an information acquisition module configured to generate air quality information according to the detection result of the air quality detection device.

The air quality detection module includes:
a time detection sub-module configured to detect whether a rotation time of the fan reaches a predetermined length of time; and
an air quality detection sub-module configured to detect the air quality by the air quality detection device when the time detection sub-module detects that the rotation time reaches the predetermined length of time.

The rotation control module includes:
a speed detection sub-module configured to detect whether an airflow speed in a current environment reaches a predetermined speed; and
a rotation control sub-module configured to control the fan to rotate when the speed detection sub-module detects that the airflow speed does not reach the predetermined speed.

Optionally, the apparatus further includes:
a signal receiving module configured to receive a trigger signal sent by a mobile terminal for triggering the detection of the air quality; and
the rotation control module configured to perform an operation of controlling the fan to rotate after the signal receiving module receives the trigger signal.

Optionally, the apparatus further includes:
a first sending module configured to directly send the air quality information to the mobile terminal which is used for displaying the air quality information;
   and/or,
a second sending module configured to send the air quality information to a server which is used for forwarding the air quality information to the mobile terminal for displaying the air quality information;
   and/or,
a third sending module configured to send the air quality information to a home gateway device which is used for forwarding the air quality information to the mobile terminal for displaying the air quality information.

Optionally, the apparatus further includes:
a time detection module configured to detect whether a predetermined time is reached, the predetermined time being a time determined at a predetermined interval, or, the predetermined time being a detection time set by a user; and
the rotation control module configured to perform an operation of controlling the fan to rotate when the time detection module detects that the predetermined time is reached.

Optionally, the apparatus further includes:
a fourth sending module configured to send the air quality information to a server which is used for forwarding all of the air quality information received before or the air quality information received last time to a mobile terminal after receiving a trigger signal sent by the mobile terminal for displaying the received air quality information;
   and/or,
a fifth sending module configured to send the air quality information to a home gateway device which is used for forwarding all of the air quality information received before or the air quality information received last time to a mobile terminal after receiving a trigger signal sent by the mobile terminal for displaying the received air quality information.

According to a third aspect of the present invention, there is provided a device as defined in claim 11.

The technical solutions provided by the embodiments of the present disclosure may include the following advantageous effects:

By controlling the fan in the household appliance to rotate, and detecting the air quality by the air quality detection device in the household appliance after the air circulates in the environment in which the household appliance is located, so as to obtain the air quality information, the problem in the related art that the measured air quality is inaccurate may be solved, and it may achieve the effects that the content of the dust particles may be accurately measured since the air quality is detected when the air circulates in its surrounding environment, and the accuracy of the measured air quality may be improved.

It is to be understood that both the foregoing general description and the following detailed description are exemplary and explanatory only and are not restrictive of the invention, as claimed.

### BRIEF DESCRIPTION OF THE DRAWINGS

The accompanying drawings, which are incorporated in and constitute a part of this specification, illustrate embodiments consistent with the disclosure and, together with the description, serve to explain the principles of the present disclosure.
Fig. 1 is a diagram showing an implement environment involved in a method for acquiring air quality according to part of exemplary embodiments.
Fig. 2 is a flow chart showing a method for acquiring air quality according to an exemplary embodiment.
Fig. 3A is a flow chart showing a method for acquiring air quality according to another exemplary embodiment.
Fig. 3B is a diagram showing a display when a mobile terminal receives a trigger signal according to another exemplary embodiment.
Fig. 3C is a diagram showing a mobile terminal displaying air quality information according to another exemplary embodiment.
Fig. 3D is a diagram showing a display of a mobile terminal when an air purifier detects air quality according to another exemplary embodiment.
Fig. 4 is a flow chart showing a method for acquiring air quality according to a further exemplary embodiment.
Fig. 5 is a block diagram showing an apparatus for acquiring air quality according to an exemplary embodiment.
Fig. 6 is a block diagram showing an apparatus for acquiring air quality according to another exemplary embodiment.
Fig. 7 is a block diagram showing a device for acquiring air quality according to an exemplary embodiment.

### DESCRIPTION OF THE EMBODIMENTS

Reference will now be made in detail to exemplary embodiments, examples of which are illustrated in the accompanying drawings. The following description refers to the accompanying drawings in which the same numbers in different drawings represent the same or similar elements unless otherwise represented. The implementations set forth in the following description of exemplary embodiments do not represent all implementations consistent with the invention. Instead, they are merely examples of apparatuses and methods consistent with aspects related to the invention as recited in the appended claims.

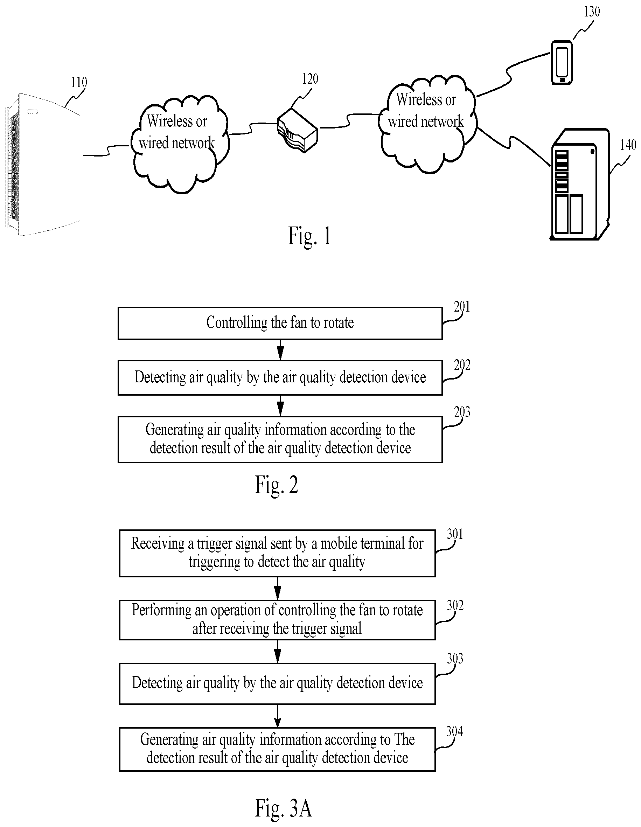

Referring to Fig. 1, it shows a structure diagram of an implement environment involved in each embodiment of the present disclosure. As shown in Fig. 1, the implement environment may include a household appliance 110, a home gateway device 120, a mobile terminal 130 and a server 140.

The household appliance 110 may include a fan and an air quality detection device. Herein, the fan is used to drive airflow in the environment which the household appliance is in, and the air quality detection device is used to detect the air quality in its surrounding environment. The fan may be a fan originally arranged in the household appliance 110, and may also be a fan specially arranged at a nearby location of the air quality detection device. The household appliance 110 may be connected with the home gateway device 120 through a wired or wireless network, and connect to the mobile terminal 130 and the server 140 via the home gateway device 120. In an actual implementation, the household appliance 110 may be an air purifier or an air quality detector.

A client is installed in the mobile terminal 130 and provided by a service provider, and a user may control the household appliance 110 through the client. In an actual implementation, when the mobile terminal 130 and the household appliance 110 are in the same local area network (LAN), the mobile terminal 130 may find the household appliance 110 through the home gateway device 120, and establish a binding relationship with the household appliance 110 which may bind with one or more mobile terminals 130.

The server 140 is a background server provided by the service provider, and is used to cooperate with the client in the mobile terminal 130 to provide services provided by the service provider for the user.

Fig. 2 is a flow chart showing a method for acquiring air quality according to an exemplary embodiment. As shown in Fig. 2, the method for acquiring air quality is applied to the household appliance 110 shown in Fig. 1, and may include the following steps.

In step 201, the fan is controlled to rotate.

In step 202, air quality is detected through the air quality detection device.

In step 203, air quality information is generated according to the detection result of the air quality detection device.

In conclusion, the method for acquiring air quality provided by the embodiments of the present disclosure may: control the fan in the household appliance to rotate, and then detect the air quality by the air quality detection device in the household appliance after the air flows in the environment in which the household appliance is located so as to obtain the air quality information; the problem in the related art that the measured air quality is inaccurate may be solved; and it achieves the effects that the content of the dust particles may be accurately measured since the air quality is detected when the air circulates in its surrounding environment, and the accuracy of the measured air quality may be improved are achieved.

In an actual implementation, the household appliance may detect the air quality in the following two situations:
A first situation,
it is detected after a trigger signal sent by the mobile terminal for triggering the detection of the air quality is received.
A second situation,
it is detected when a predetermined time is reached, wherein the predetermined time is a time determined at a predetermined interval, or, a detection time set by the user.

Therefore, the above two situations will be explained in detail respectively in the following two exemplary embodiments.

Fig. 3A is a flow chart showing a method for acquiring air quality according to an exemplary embodiment. The present embodiment takes that the method for acquiring air quality is applied to the household appliance 110 shown in Fig. 1 and the household appliance detects the air quality in the above first situation, as an example. As shown in Fig. 3A, the method for acquiring air quality may include the following steps.

In step 301, a trigger signal sent by a mobile terminal for triggering the detection of the air quality is received.

The present embodiment takes that the household appliance is an air purifier, as an example. When the user wants to view the air quality, the user may operate the mobile terminal, so that the mobile terminal sends the trigger signal for triggering the detection of the air quality to the air purifier. The air purifier may correspondingly receive the trigger signal.

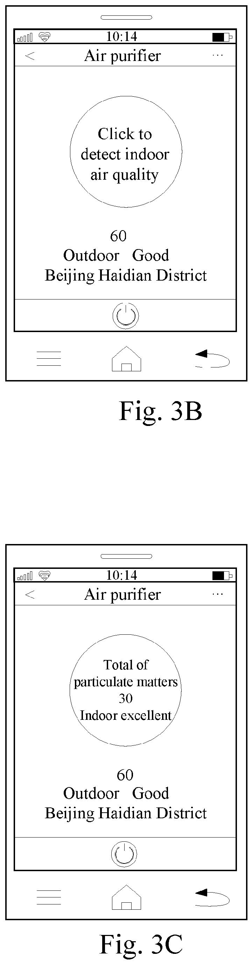

For example, when a user Xiaoming wants to view air quality in his home, Xiaoming may open a client installed in a mobile phone for providing services to the air purifier. Referring to Fig. 3B, Xiaoming may click "detect indoor air quality" in the client, and the mobile phone may send the trigger signal for triggering the detection of the air quality to the air purifier after receiving a clicking signal. Correspondingly, the air purifier may receive the trigger signal sent by the mobile phone.

In step 302, an operation of controlling the fan to rotate is performed after the trigger signal is received.

The air purifier may control the fan in the air purifier to rotate after the air purifier receives the trigger signal. The fan for driving airflow in the environment in which the household appliance is located, may be an original fan in the air purifier, and may also be a new added fan located before the air quality detection device. The present embodiment is not limited thereto.

The air purifier may start up a motor of the fan after receiving the trigger signal, and the fan is driven to rotate through the rotation of the motor.

In step 303, air quality is detected by the air quality detection device.

The air purifier may detect the air quality in the environment by its internal air quality detection device after the fan rotates and then the air in the environment in which the air purifier is located begins to circulate.

In step 304, air quality information is generated according to the detection result of the air quality detection device.

The air purifier may generate the air quality information according to the detection result of the air quality detection device. Herein, the air quality information may include at least one of air quality level, AQI (Air Quality Index), the content of fine particles, the content of inhalable particles, the content of sulfur dioxide, the content of nitrogen dioxide, the content of ozone and the content of carbonic oxide.

The air purifier may feed back the received air quality information to the user after obtaining the air quality information. In an actual implementation, the air purifier may feed back through at least one of the following manners.

A first manner,
the air quality information is directly sent to the mobile terminal which is used for displaying the air quality information.

The air purifier may send the air quality information to the mobile terminal. The mobile terminal displays the air quality information after receiving the air quality information.

A second manner,
the air quality information is sent to a server which is used for forwarding the air quality information to the mobile terminal for displaying the air quality information.

The air purifier may also send the obtained air quality information to the server, and then the air quality information is forwarded to the mobile terminal by the server. The mobile terminal receives the air quality information sent by the server, and displays the air quality information.

The server may be a background server for providing services to the client. However, a client may be installed in the mobile terminal, and thus the server may send the air quality information to the mobile terminal according to account information of the client. The present embodiment is not limited thereto.

A third manner,
the air quality information is sent to a home gateway device which is used for forwarding the air quality information to the mobile terminal for displaying the air quality information.

Since the mobile terminal may access information in the home gateway device, the air purifier may also send the obtained air quality information to the home gateway device, and the air quality information is forwarded to the mobile terminal by the home gateway device. The mobile terminal correspondingly receives the air quality information, and displays the air quality information.

For example, assuming that the air quality information including the air quality level and the total of particulate matters, and the air quality level is best, the total of particulate matters is 30, the mobile phone may display a display interface as shown in Fig. 3C after receiving the air quality information. As shown in Fig. 3C, the mobile phone may also display outdoor air quality information while the mobile phone displays the air quality information measured by the air purifier. The outdoor air quality information may be information acquired by the mobile phone from the server, and the present embodiment is not limited thereto.

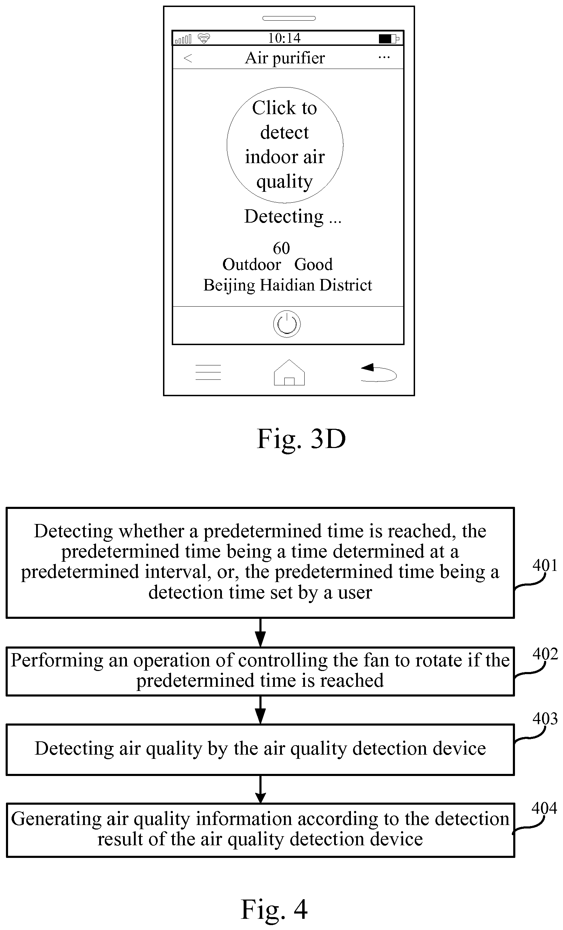

It should be additionally explained that, after the mobile terminal sends the trigger signal to the air purifier, and the mobile terminal displays the air quality information, the mobile terminal may display the display interface for showing that the air purifier is detecting the air quality. For example, still taking the above example as an example, the mobile phone may display an interface as shown in Fig. 3D after Xiaoming clicks 'detect the indoor air quality' in Fig. 3B. Then, the mobile phone sends the trigger signal to the air purifier, and may display the interface shown in Fig. 3C when receiving the air quality information.

It should be another additionally explained that, the present embodiment only takes that the user sends the trigger signal to the air purifier through the mobile terminal, as an example, In an actual implementation, since a switch used to trigger air quality detection may be arranged in the air purifier, the user may also directly trigger the air quality detection through the switch on the air purifier, that is, the air purifier may directly receive the trigger signal applied by the user for turning on the switch. The present embodiment is not limited thereto.

It should be further additionally explained that, an indicator light for representing the air quality may arranged in the air purifier, and thus the air purifier may also feed back a measurement result to the user through the indicator light after the air quality information is measured by the air purifier. For example, a green light represents that the air quality is excellent, a yellow light represents that the air quality is good, and a red light represents the air quality is polluted. The present embodiment is not limited thereto. And, in an actual implementation, when a display screen is arranged in the air purifier, the air purifier may also display the air quality information to the user through the display screen, and the present embodiment is not limited thereto.

In conclusion, the method for acquiring air quality provided by the embodiments of the present disclosure may control the fan in the household appliance to rotate, and then detect the air quality by the air quality detection device in the household appliance after the air flows in the environment in which the household appliance is located, so as to obtain the air quality information. Thus, the problem in the related art that the measured air quality is inaccurate is solved, and it may achieve the effects that the content of the dust particles may be accurately measured since the air quality is detected when the air circulates in its surrounding environment, and the accuracy of the measured air quality may be improved.

Fig. 4 is a flow chart showing a method for acquiring air quality according to an exemplary embodiment. The present embodiment takes that the method for acquiring air quality is applied to the household appliance 110 shown in Fig. 1 and the household appliance detects the air quality in the case of the above second case, as an example. As shown in Fig. 4, the method for acquiring air quality may include the following steps.

In step 401, whether a predetermined time is reached is detected, the predetermined time being a time determined at a predetermined interval, or, the predetermined time being a detection time set by a user.

The present embodiment still takes that the household appliance is an air purifier, as an example. The air purifier may detect whether the predetermined time is reached in real time.

Herein, the predetermined time is a time determined at a predetermined interval. The predetermined interval may be a default time interval in the air purifier; and may also be a time interval preset by the user. For example, the user sets 'detect indoor air quality on the hour' through the mobile phone in advance, and sends the setting information to the air purifier. In this case, the predetermined time is time on the hour, such as 'one o'clock, two o'clock, three o'clock', and the like.

Or , the predetermined time may also be a detection time set by the user. For example, the user sets 'detect indoor air quality at 5:30 pm', and then the predetermined time is 5:30 pm set by the user.

In step 402, an operation of controlling the fan to rotate is performed if the predetermined time is reached.

The air purifier may control the fan to rotate after the detection result of the air purifier is that the predetermined time is reached. The fan, which is used for driving airflow in the environment in which the household appliance is located, may be an original fan of the air purifier, and may also be a new added fan located before the air quality detection device. The present embodiment is not limited thereto.

The air purifier may start up a motor of the fan, and the fan is driven to rotate through the rotation of the motor.

In step 403, air quality is detected through the air quality detection device.

The air purifier may detect the air quality in the environment through its internal air quality detection device after the fan rotates and then the air in the environment in which the air purifier is located begins to flow.

In step 404, air quality information is generated according to the detection result of the air quality detection device.

The air purifier may generate the air quality information according to the detection result of the air quality detection device. Herein, the air quality information may include at least one of air quality level, AQI, the content of fine particles, the content of inhalable particles, the content of sulfur dioxide, the content of nitrogen dioxide, the content of ozone and the content of carbonic oxide.

The air purifier may feed back the received air quality information to the user after the air purifier obtains the air quality information. In an actual implementation, the air purifier may feed back in at least one of the following manners.

A first manner,
the air quality information is sent to a server which is used for forwarding all of the air quality information received before or the air quality information received last time to the mobile terminal after receiving a trigger signal sent by a mobile terminal for displaying the received air quality information.

The air purifier may send the air quality information to the server after the air purifier obtains the air quality information. The server is used to forward all of the air quality information received before or the air quality information received last time to the mobile terminal after receiving a trigger signal sent by a mobile terminal for displaying the received air quality information.

The mobile terminal is a terminal installed with the client, and the server may be a background server for providing services to the client. The mobile terminal may send the trigger signal to the server, and the server forwards the received air quality information sent by the air purifier to the mobile terminal after receiving the trigger signal. Or, the server forwards the air quality information received last time to the mobile terminal after receiving the trigger signal. Herein, the trigger signal may be a trigger signal sent by the mobile terminal after the user performs a long-press operation to an information acquisition button in the client. The present embodiment is not limited thereto.

The steps that mobile terminal sends the trigger signal to the server may include at least one of the following possible implementations.
(1) The trigger signal is automatically sent to the server when a screen of the mobile terminal is lit.
   The mobile terminal may automatically send the trigger signal to the server when the user lights a terminal screen.
(2) The trigger signal is automatically sent to the server when the screen of the mobile terminal is unlocked.
(3) The trigger signal is automatically sent to the server when the client runs in the mobile terminal.
   The mobile terminal may automatically send the trigger signal to the server when the user opens the client.
(4) The trigger signal is automatically sent to the server when the mobile terminal is connected to a network.

The mobile terminal displays the air quality information after receiving the air quality information.

A second manner,
the air quality information is sent to a home gateway device which is used for forwarding all of the air quality information received before or the air quality information received last time to the mobile terminal after receiving a trigger signal sent by a mobile terminal for displaying the received air quality information.

Similar to the above implementation, the air purifier may also send the measured air quality information to the home gateway device. The home gateway device is used to forward all of the air quality information received before or the air quality information received last time to the mobile terminal after receiving a trigger signal sent by a mobile terminal for displaying the received air quality information, and the present embodiment is not limited thereto.

In conclusion, the method for acquiring air quality provided by the embodiments of the present disclosure may: control the fan in the household appliance to rotate, and then detect the air quality by the air quality detection device in the household appliance after the air flows in the environment in which the household appliance is located, so as to obtain the air quality information. Thus, the problem in the related art that the measured air quality is inaccurate is solved and it may achieve the effects that the content of the dust particles may be accurately measured since the air quality is detected when the air circulates in its surrounding environment, and the accuracy of the measured air quality may be improved.

In the present embodiment, the household appliance may directly control the fan to rotate when the household appliance reaches the predetermined timing, so that the air quality is measured, whereby the operation performed when the user acquires the air quality may be simplified.

It should be additionally explained that, in the above each embodiment, the household appliance controls the fan to rotate may include the following steps:

Firstly, whether an airflow speed in a current environment reaches a predetermined speed is detected.

The household appliance may acquire the airflow speed in the current environment, and detect whether the acquired airflow speed reaches the predetermined speed.

Secondly, the fan is controlled to rotate if the airflow speed does not reach the predetermined speed.

If the detection result of the household appliance is that the airflow speed does not reach the predetermined speed, in order to avoid the problem that the measured air quality is inaccurate due to the poor ventilation, the household appliance controls the fan to rotate. The household appliance detects the air quality after the airflow is driven by the rotation of the fan, thus improving the accuracy of the measured air quality.

However, if the airflow speed reaches the predetermined speed, the household appliance may directly detect the air quality by the air quality detection device, and the present embodiment is not limited thereto.

It should be another additionally explained that, in the above each embodiment, the steps that the household appliance detects the air quality by the air quality detection device may include the following steps:
Firstly, whether a rotation time of the fan reaches a predetermined length of time is detected.

The household appliance may count the rotation time of the fan, and detect whether the rotation time of the fan has reached the predetermined length of time.

For example, taking that the predetermined length of time is 30S, as an example, the household appliance may detect whether the rotation time of the fan reaches 30S after the household appliance controls the fan to rotate.

Secondly, the air quality is detected through the air quality detection device if the rotation time reaches the predetermined length of time.

If the detection result of the household appliance is that the rotation time reaches the predetermined length of time, it illustrates that the air has absolutely been circulated in the environment in which the household appliance is located, and at this time, the household appliance may detect the air quality by the air quality detection device.

Since the present embodiment detects the air quality after the fan rotates a certain length of time, the problem that the air quality detection device hardly accurately detects the air quality due to the airless ventilation may be avoided, thus further improving the accuracy of the measured air quality.

It should be further additionally explained that, if the household appliance is the air purifier which may purify the air, when the air quality information detected by the air purifier represents the poorer air quality, the air purifier may directly perform an operation of purifying air or send a warning (an alarm, an indicator light or a prompt tone), or the air purifier may send to the mobile terminal a prompt message for reminding a user that the current air quality is poorer and suggesting the user to open the air purifier, and then the mobile terminal displays the prompt message and controls the air purifier to purify the air when a user's confirmation signal is received. The present embodiment is not limited thereto.

The following is the embodiment of the apparatus in the present disclosure, which may be configured to execute the embodiment of the method in the present disclosure. Please refer to the embodiment of the method in the present disclosure with regard to undisclosed details about the embodiment of the apparatus in the present disclosure.

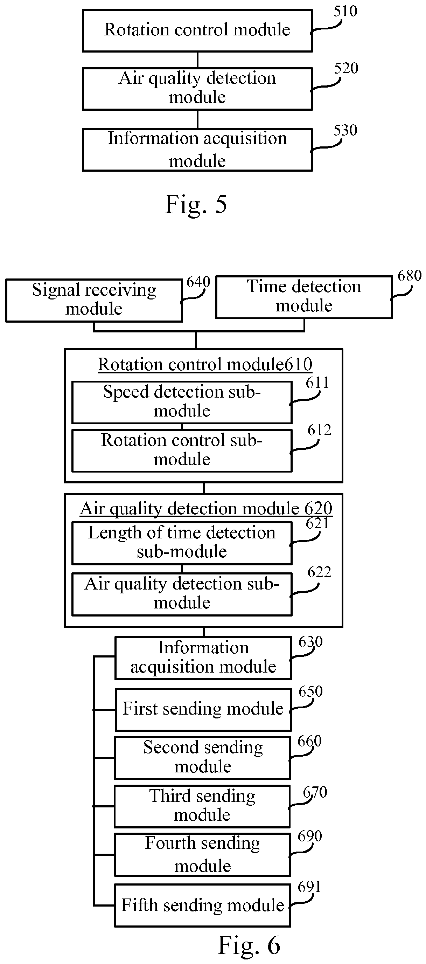

Fig. 5 is a block diagram showing an apparatus for acquiring air quality according to an exemplary embodiment. As shown in Fig. 5, the apparatus for acquiring air quality is applied in the household appliance 110, and may include but not limited to: a rotation control module 510, an air quality detection module 520 and an information acquisition module 530.

The rotation control module 510 is configured to control the fan to rotate.

The air quality detection module 520 is configured to detect air quality by the air quality detection device.

The information acquisition module 530 is configured to generate air quality information according to the detection result of the air quality detection device.

In conclusion, the apparatus for acquiring air quality provided by the embodiments of the present disclosure may: control the fan in the household appliance to rotate, and then detect the air quality by the air quality detection device in the household appliance after the air flows in the environment in which the household appliance is located, so as to obtain the air quality information. Thus, the problem in the related art that the measured air quality is inaccurate may be solved, and it may achieve the effects that the content of the dust particles may be accurately measured since the air quality is detected when the air circulates in its surrounding environment, and the accuracy of the measured air quality may be improved.

Fig. 6 is a block diagram showing an apparatus for acquiring air quality according to another exemplary embodiment. As shown in Fig. 6, the apparatus for acquiring air quality is applied in the household appliance 110. The apparatus for acquiring air quality may include but not limited to: a rotation control module 610, an air quality detection module 620 and an information acquisition module 630.

The rotation control module 610 is configured to control the fan to rotate.

The air quality detection module 620 is configured to detect air quality by the air quality detection device.

The information acquisition module 630 is configured to generate air quality information according to the detection result of the air quality detection device.

Optionally, the air quality detection module 620 includes:
a time detection sub-module 621 configured to detect whether a rotation time of the fan reaches a predetermined length of time; and
an air quality detection sub-module 622 configured to detect the air quality by the air quality detection device when the detection result of the time detection sub-module 621 is that the rotation time reaches the predetermined length of time.

Optionally, the rotation control module 610 includes:
a speed detection sub-module 611 configured to detect whether an airflow speed in a current environment reaches a predetermined speed; and
a rotation control sub-module 612 configured to control the fan to rotate when the speed detection sub-module detects that the airflow speed does not reach the predetermined speed.

Optionally, the apparatus further includes:
a signal receiving module 640 configured to receive a trigger signal sent by a mobile terminal for triggering the detection of the air quality; and
the rotation control module 610 configured to perform an operation of controlling the fan to rotate after the signal receiving module 640 receives the trigger signal.

Optionally, the apparatus further includes:
a first sending module 650 configured to directly send the air quality information to the mobile terminal which is used for displaying the air quality information;
   and/or,
a second sending module 660 configured to send the air quality information to a server which is used for forwarding the air quality information to the mobile terminal for displaying the air quality information;
   and/or,
a third sending module 670 configured to send the air quality information to a home gateway device which is used for forwarding the air quality information to the mobile terminal for displaying the air quality information.

Optionally, the apparatus further includes:
a time detection module 680 configured to detect whether a predetermined time is reached, the predetermined time being a time determined at a predetermined interval, or, the predetermined time being a detection time set by a user; and
the rotation control module 610 configured to perform an operation of controlling the fan to rotate when the time detection module detects that the predetermined time is reached.

Optionally, the apparatus further includes:
a fourth sending module 690 configured to send the air quality information to a server which is used for forwarding all of the air quality information received before or the air quality information received last time to a mobile terminal after receiving a trigger signal sent by the mobile terminal for displaying the received air quality information;
   and/or,
a fifth sending module 691 configured to send the air quality information to a home gateway device which is used for forwarding all of the air quality information received before or the air quality information received last time to the mobile terminal after receiving a trigger signal sent by a mobile terminal for displaying the received air quality information.

In conclusion, the apparatus for acquiring air quality provided by the embodiments of the present disclosure may: control the fan in the household appliance to rotate, and then detect the air quality by the air quality detection device in the household appliance after the air flows in the environment in which the household appliance is located so as to obtain the air quality information. Thus, the problem in the related art that the measured air quality is inaccurate is solved; and it may achieve the effects that the content of the dust particles may be accurately measured since the air quality is detected when the air circulates in its surrounding environment, and the accuracy of the measured air quality may be improved.

With respect to the apparatus in the above embodiments, the specific manners for performing operations for individual modules therein have been described in detail in the embodiments regarding the methods, which will not be elaborated herein.

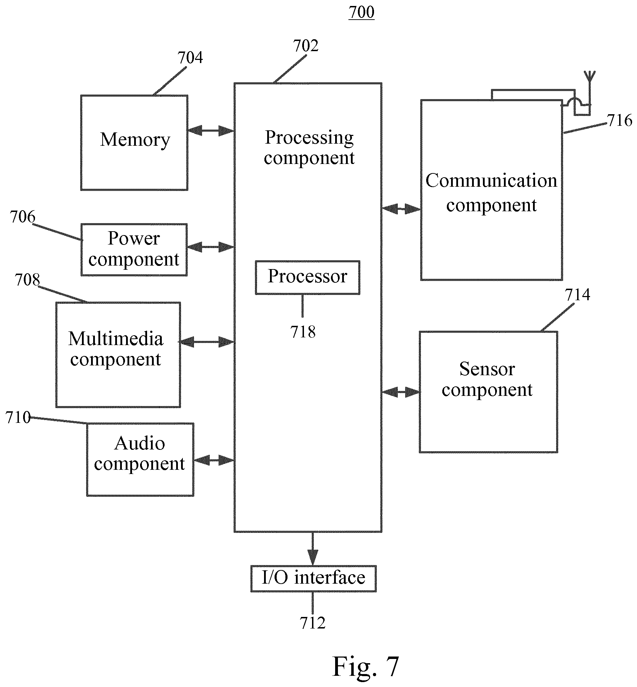

Fig. 7 is a block diagram showing a device 700 for acquiring air quality according to an exemplary embodiment. For example, the device 700 may be a mobile phone, a computer, a digital broadcast terminal, a messaging device, a gaming console, a tablet, a medical device, exercise equipment, a personal digital assistant (PDA), and the like.

Referring to Fig. 7, the device 700 may include one or more of the following components: a processing component 702, a memory 704, a power component 706, a multimedia component 708, an audio component 710, an input/output (I/O) interface 712, a sensor component 714, and a communication component 716.

The processing component 702 usually controls overall operations of the device 700, such as the operations associated with display, telephone calls, data communications, camera operations, and recording operations. The processing component 702 may include one or more processors 718 to execute instructions to perform all or part of the steps in the above described methods. Moreover, the processing component 702 may include one or more modules which facilitate the interaction between the processing component 702 and other components. For instance, the processing component 702 may include a multimedia module to facilitate the interaction between the multimedia component 708 and the processing component 702.

The memory 704 is configured to store various types of data to support the operation of the device 700. Examples of such data include instructions for any application or method operated on the device 700, contact data, phonebook data, messages, pictures, videos, etc. The memory 704 may be implemented using any type of volatile or non-volatile memory device or combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, a flash memory, a magnetic or optical disk.

The power component 706 provides power to various components of the device 700. The power component 706 may include a power management system, one or more power sources, and other components associated with the generation, management, and distribution of power in the device 700.

The multimedia component 708 includes a screen providing an output interface between the device 700 and the user. In some embodiments, the screen may include a liquid crystal display (LCD) and a touch panel (TP). If the screen includes the touch panel, the screen may be implemented as a touch screen to receive input signals from the user. The touch panel includes one or more touch sensors to sense touches, slips, and gestures on the touch panel. The touch sensors may not only sense a boundary of a touch or slip action, but also sense a period of time and a pressure associated with the touch or slip action. In some embodiments, the multimedia component 708 includes a front camera and/or a rear camera. The front camera and/or the rear camera may receive an external multimedia datum while the device 700 is in an operation manner, such as a photographing manner or a video manner. Each of the front camera and the rear camera may be a fixed optical lens system or have focus and optical zoom capability.

The audio component 710 is configured to output and/or input audio signals. For example, the audio component 710 includes a microphone (MIC) configured to receive an external audio signal when the device 700 is in an operation manner, such as a call manner, a recording manner, and a voice identification manner. The received audio signal may be further stored in the memory 704 or transmitted via the communication component 716. In some embodiments, the audio component 710 further includes a speaker to output audio signals.

The I/O interface 712 provides an interface between the processing component 702 and peripheral interface modules, such as a keyboard, a click wheel, a button, and the like. The button may include, but not limited to, a home button, a volume button, a starting button, and a locking button.

The sensor component 714 includes one or more sensors to provide status assessments of various aspects of the device 700. For instance, the sensor component 714 may detect an open/closed status of the device 700, relative positioning of components, e.g., the display and the keyboard, of the device 700, a change in position of the device 700 or a component of the device 700, a presence or absence of user contact with the device 700, an orientation or an acceleration/deceleration of the device 700, and a change in temperature of the device 700. The sensor component 714 may include a proximity sensor configured to detect the presence of nearby objects without any physical contact. The sensor component 714 may also include a light sensor, such as a CMOS or CCD image sensor, for use in imaging applications. In some embodiments, the sensor component 714 may also include an accelerometer sensor, a gyroscope sensor, a magnetic sensor, a pressure sensor, or a temperature sensor.

The communication component 716 is configured to facilitate communication, wired or wirelessly, between the device 700 and other devices. The device 700 may access a wireless network based on a communication standard, such as WiFi, 2G, or 3G, or a combination thereof. In one exemplary embodiment, the communication component 716 receives a broadcast signal or broadcast associated information from an external broadcast management system via a broadcast channel. In one exemplary embodiment, the communication component 716 further includes a near field communication (NFC) module to facilitate short-range communications. For example, the NFC module may be implemented based on a radio frequency identification (RFID) technology, an infrared data association (IrDA) technology, an ultra-wideband (UWB) technology, a Bluetooth (BT) technology, and other technologies.

In exemplary embodiments, the device 700 may be implemented with one or more application specific integrated circuits (ASICs), digital signal processors (DSPs), digital signal processing devices (DSPDs), programmable logic devices (PLDs), field programmable gate arrays (FPGAs), controllers, micro-controllers, microprocessors, or other electronic components, for performing the above method.

In exemplary embodiments, there is also provided a non-transitory computer readable storage medium including instructions, such as included in the memory 704, executable by the processor 718 in the device 700, for performing the above method. For example, the non-transitory computer-readable storage medium may be a ROM, a random access memory (RAM), a CD-ROM, a magnetic tape, a floppy disc, an optical data storage device, and the like.

## Claims

1. A method for acquiring air quality applied in a household appliance comprising a fan and an air quality detection device, and comprises:
controlling (201) the fan to rotate;
detecting (202) air quality by the air quality detection device; and
generating (203) air quality information according to the detection result of the air quality detection device,
**characterized in that** controlling the fan to rotate comprises:
detecting whether an airflow speed in a current environment reaches a predetermined speed; and
controlling the fan to rotate if the airflow speed does not reach the predetermined speed,
and **in that** detecting the air quality by the air quality detection device comprises:
detecting whether a rotation time of the fan reaches a predetermined length of time; and
detecting the air quality by the air quality detection device if the rotation time reaches the predetermined length of time.

2. The method according to claim 1, **characterized in that**, the method further comprises:
receiving (301) a trigger signal sent by a mobile terminal for triggering the detection of the air quality; and
performing (302) an operation of controlling the fan to rotate after receiving the trigger signal.

3. The method according to claim 2, **characterized in that**, the method further comprises:
directly sending the air quality information to the mobile terminal which is used for displaying the air quality information;
and/or,
sending the air quality information to a server which is used for forwarding the air quality information to the mobile terminal for displaying the air quality information;
and/or,
sending the air quality information to a home gateway device which is used for forwarding the air quality information to the mobile terminal for displaying the air quality information.

4. The method according to claim 1, **characterized in that**, the method further comprises:
detecting (401) whether a predetermined time is reached, the predetermined time being a time determined at a predetermined interval, or, the predetermined time being a detection time set by a user; and
performing (402) an operation of controlling the fan to rotate if the predetermined time is reached.

5. The method according to claim 4, **characterized in that**, the method further comprises:
sending the air quality information to a server, which is used for forwarding all of the air quality information received before or the air quality information received last time to a mobile terminal after receiving a trigger signal sent by the mobile terminal for displaying the received air quality information;
and/or,
sending the air quality information to a home gateway device which is used for forwarding all of the air quality information received before or the air quality information received last time to a mobile terminal after receiving a trigger signal sent by the mobile terminal for displaying the received air quality information.

6. An apparatus for acquiring air quality for a household appliance comprising a fan and an air quality detection device, the apparatus comprising:
a rotation control module (510) configured to control the fan to rotate;
an air quality detection module (520) configured to detect air quality by the air quality detection device; and
an information acquisition module (530) configured to generate air quality information according to the detection result of the air quality detection device,
**characterized in that** the rotation control module comprises:
a speed detection sub-module (611) configured to detect whether an airflow speed in a current environment reaches a predetermined speed; and
a rotation control sub-module (612) configured to control the fan to rotate when the speed detection sub-module (611) detects that the airflow speed does not reach the predetermined speed,
and **in that** the air quality detection module comprises:
a time detection sub-module (621) configured to detect whether a rotation time of the fan reaches a predetermined length of time ; and
an air quality detection sub-module (622) configured to detect the air quality by the air quality detection device when the detection result of the time detection sub-module (621) is that the rotation time reaches the predetermined length of time.

7. The apparatus according to claim 6, **characterized in that**, the apparatus further comprises:
a signal receiving module (640) configured to receive a trigger signal sent by a mobile terminal for triggering the detection of the air quality; and
the rotation control module (510) configured to perform an operation of controlling the fan to rotate after the signal receiving module receives the trigger signal.

8. The apparatus according to claim 7, **characterized in that**, the apparatus further comprises:
a first sending module (650) configured to directly send the air quality information to the mobile terminal which is used for displaying the air quality information;
and/or,
a second sending module (660) configured to send the air quality information to a server which is used for forwarding the air quality information to the mobile terminal for displaying the air quality information;
and/or,
a third sending module (670) configured to send the air quality information to a home gateway device which is used for forwarding the air quality information to the mobile terminal which is used for displaying the air quality information.

9. The apparatus according to claim 6, **characterized in that**, the apparatus further comprises:
a time detection module (680) configured to detect whether a predetermined time is reached, the predetermined time being a time determined at a predetermined interval, or, the predetermined time being a detection time set by a user; and
the rotation control module (610) configured to perform an operation of controlling the fan to rotate when the time detection module detects that the predetermined time is reached.

10. The apparatus according to claims 8 and 9, **characterized in that**, the apparatus further comprises:
a fourth sending module (690) configured to send the air quality information to a server which is used for forwarding all of the air quality information received before or the air quality information received last time to a mobile terminal after receiving a trigger signal sent by the mobile terminal for displaying the received air quality information;
and/or,
a fifth sending module (691) configured to send the air quality information to a home gateway device which is used for forwarding all of the air quality information received before or the air quality information received last time to a mobile terminal after receiving a trigger signal sent by the mobile terminal for displaying the received air quality information.

11. A device for acquiring air quality for a household appliance comprising a fan and an air quality detection device, the device for acquiring air quality comprising:
a processor; and
a memory for storing instructions executable by the processor;
wherein the processor is configured to perform:
controlling the fan to rotate;
detecting air quality by the air quality detection device;
generating air quality information according to the detection result of the air quality detection device,
**characterized in that** the processor is further configured to perform:
detecting whether an airflow speed in a current environment reaches a predetermined speed;
controlling the fan to rotate if the airflow speed does not reach the predetermined speed;
detecting whether a rotation time of the fan reaches a predetermined length of time;
detecting the air quality by the air quality detection device if the rotation time reaches the predetermined length of time.

12. A computer program, which when executing on a processor (718), performs the method according to any one of claims 1 to 5.

## Patentansprüche

1. Verfahren zum Erlangen von Luftqualität, das in einem Haushaltsgerät angewendet wird, umfassend einen Lüfter und eine Luftqualitätserfassungsvorrichtung, und umfassend:
Steuern bzw. Regeln (201) des Lüfters, sich zu drehen;
Erfassen (202) der Luftqualität durch die Luftqualitätserfassungsvorrichtung; und
Erzeugen (203) von Luftqualitätsinformationen gemäß dem Erfassungsergebnis der Luftqualitätserfassungsvorrichtung,
**dadurch gekennzeichnet, dass** das Steuern bzw. Regeln des Lüfters umfasst:
Erfassen, ob eine Luftströmungsgeschwindigkeit in einer gegenwärtigen Umgebung eine vorbestimmte Geschwindigkeit erreicht; und
Steuern bzw. Regeln des Lüfters, sich zu drehen, wenn die Luftströmungsgeschwindigkeit die vorbestimmte Geschwindigkeit nicht erreicht,
und darin, dass das Erfassen der Luftqualität durch die Luftqualitätserfassungsvorrichtung umfasst:
Erfassen, ob eine Drehzeit des Lüfters eine vorbestimmte Dauer erreicht; und
Erfassen der Luftqualität durch die Luftqualitätserfassungsvorrichtung, wenn die Drehzeit die vorbestimmte Dauer erreicht.

2. Verfahren nach Anspruch 1, **dadurch gekennzeichnet, dass** das Verfahren weiter umfasst:
Empfangen (301) eines Auslösesignals, das von einem mobilen Endgerät zum Auslösen der Erfassung der Luftqualität gesendet wurde; und
Durchführen (302) eines Steuerungs- bzw. Regelungsvorgangs des Lüfters, sich zu drehen, nachdem das Auslösesignal empfangen wurde.

3. Verfahren nach Anspruch 2, **dadurch gekennzeichnet, dass** das Verfahren weiter umfasst:
Direktes Senden der Luftqualitätsinformationen an das mobile Endgerät, das zum Anzeigen der Luftqualitätsinformationen genutzt wird;
und/oder
Senden der Luftqualitätsinformationen an einen Server, der zum Weiterleiten der Luftqualitätsinformationen an das mobile Endgerät zum Anzeigen der Luftqualitätsinformationen genutzt wird;
und/oder
Senden der Luftqualitätsinformationen an eine Heimgateway-Vorrichtung, die zum Weiterleiten der Luftqualitätsinformationen an das mobile Endgerät zum Anzeigen der Luftqualitätsinformationen genutzt wird.

4. Verfahren nach Anspruch 1, **dadurch gekennzeichnet, dass** das Verfahren weiter umfasst:
Erfassen (401), ob eine vorbestimmte Zeit erreicht wurde, wobei die vorbestimmte Zeit eine Zeit ist, die in einem vorbestimmten Abstand bestimmt wird, wobei die vorbestimmte Zeit eine durch den Nutzer eingestellte Erfassungszeit ist; und
Durchführen (402) eines Steuerungs- bzw. Regelungsvorgangs des Lüfters, sich zu drehen, wenn die vorbestimmte Zeit erreicht ist.

5. Verfahren nach Anspruch 4, **dadurch gekennzeichnet, dass** das Verfahren weiter umfasst:
Senden der Luftqualitätsinformationen an einen Server, der genutzt wird, um alle der zuvor empfangenen Luftqualitätsinformationen oder die beim letzten Mal empfangenen Luftqualitätsinformationen an ein mobiles Endgerät zu senden, nachdem ein Auslösesignal empfangen wurde, das durch das mobile Endgerät zum Anzeigen der Luftqualitätsinformationen gesendet wurde;
und/oder
Senden der Luftqualitätsinformationen an eine Heimgateway-Vorrichtung, die genutzt wird, um alle der zuvor empfangenen Luftqualitätsinformationen oder die beim letzten Mal empfangenen Luftqualitätsinformationen an ein mobiles Endgerät zu senden, nachdem ein Auslösesignal empfangen wurde, das durch das mobile Endgerät zum Anzeigen der Luftqualitätsinformationen gesendet wurde.

6. Apparat zum Erlangen von Luftqualität für ein Haushaltsgerät, umfassend einen Lüfter und eine Luftqualitätserfassungsvorrichtung, wobei der Apparat umfasst:
ein Drehsteuerungs- bzw. Regelungsmodul (510), konfiguriert, um zu steuern bzw. zu regeln, dass der Lüfter sich dreht;
ein Luftqualitätserfassungsmodul (520), konfiguriert, um die Luftqualität durch die Luftqualitätserfassungsvorrichtung zu erfassen; und
ein Informationsbeschaffungsmodul (530), konfiguriert, um Luftqualitätsinformationen gemäß dem Erfassungsergebnis der Luftqualitätserfassungsvorrichtung zu erzeugen,
**dadurch gekennzeichnet, dass** das Drehsteuerungs- bzw. Regelungsmodul umfasst:
ein Geschwindigkeitserfassungsuntermodul (611), konfiguriert, um zu erfassen, ob eine Luftströmungsgeschwindigkeit in einer gegenwärtigen Umgebung eine vorbestimmte Geschwindigkeit erreicht; und
ein Drehsteuerungs- bzw. -Regelungsuntermodul (621), konfiguriert, um zu steuern bzw. regeln, dass der Lüfter sich dreht, wenn das Geschwindigkeitserfassungsuntermodul (611) erfasst, dass die Luftströmungsgeschwindigkeit nicht die vorbestimmte Geschwindigkeit erreicht,
und darin, dass das Luftqualitätserfassungsmodul umfasst:
ein Zeiterfassungsuntermodul (621), konfiguriert, um zu erfassen, ob eine Drehzeit des Lüfters eine vorbestimmte Dauer erreicht; und
ein Luftqualitätserfassungsuntermodul (622), konfiguriert, um die Luftqualität durch die Luftqualitätserfassungsvorrichtung zu erfassen, wenn das Erfassungsergebnis des Zeiterfassungsuntermoduls (621) ist, dass die Drehzeit die vorbestimme Dauer erreicht.

7. Apparat nach Anspruch 6, **dadurch gekennzeichnet, dass** der Apparat weiter umfasst:
ein Signalempfangsmodul (640), konfiguriert, um ein Auslösesignal zu empfangen, das durch ein mobiles Endgerät zum Auslösen der Luftqualitätserfassung gesendet wurde; und
das Drehsteuerungs- bzw. Regelungsmodul (510), konfiguriert, um einen Steuerungs- bzw. Regelungsvorgangs des Lüfters, sich zu drehen, nachdem das Auslösesignal empfangen wurde, durchzuführen.

8. Apparat nach Anspruch 7, **dadurch gekennzeichnet, dass** der Apparat weiter umfasst:
ein erstes Sendemodul (650), konfiguriert, um direkt die Luftqualitätsinformationen an das mobile Endgerät zu senden, das zum Anzeigen der Luftqualitätsinformationen genutzt wird;
und/oder
ein zweites Sendemodul (660), konfiguriert, um die Luftqualitätsinformationen an einen Server zu senden, der zum Weiterleiten der Luftqualitätsinformationen an das mobile Endgerät zum Anzeigen der Luftqualitätsinformationen genutzt wird;
und/oder
ein drittes Sendemodul (670), konfiguriert, um die Luftqualitätsinformationen an eine Heimgateway-Vorrichtung zu senden, die zum Weiterleiten der Luftqualitätsinformationen an das mobile Endgerät zum Anzeigen der Luftqualitätsinformationen genutzt wird.

9. Apparat nach Anspruch 6, **dadurch gekennzeichnet, dass** der Apparat weiter umfasst:
ein Zeiterfassungsmodul (680), konfiguriert, um zu erfassen, ob eine vorbestimmte Zeit erreicht wird, wobei die vorbestimmte Zeit eine Zeit ist, die in einem vorbestimmten Abstand bestimmt wird, oder wobei die vorbestimmte Zeit eine durch den Nutzer eingestellte Erfassungszeit ist; und
das Drehsteuerungs- bzw. Regelungsmodul (610), konfiguriert, um einen Steuerungs- bzw. Regelungsvorgang des Lüfters, sich zu drehen, durchzuführen, wenn das Zeiterfassungsmodul erfasst, dass die vorbestimmte Zeit erreicht ist.

10. Apparat nach Ansprüchen 8 und 9, **dadurch gekennzeichnet, dass** der Apparat weiter umfasst:
ein viertes Sendemodul (690), konfiguriert, um die Luftqualitätsinformationen an einen Server zu senden, der genutzt wird, um alle der zuvor empfangenen Luftqualitätsinformationen oder die beim letzten Mal empfangenen Luftqualitätsinformationen an ein mobiles Endgerät zu senden, nachdem ein Auslösesignal empfangen wurde, das durch das mobile Endgerät
zum Anzeigen der Luftqualitätsinformationen gesendet wurde;
und/oder
ein fünftes Sendemodul (691), konfiguriert, um die Luftqualitätsinformationen an eine Heimgateway-Vorrichtung zu senden, die genutzt wird, um alle der zuvor empfangenen Luftqualitätsinformationen oder die beim letzten Mal empfangenen Luftqualitätsinformationen an ein mobiles Endgerät zu senden, nachdem ein Auslösesignal empfangen wurde, das durch das mobile Endgerät
zum Anzeigen der Luftqualitätsinformationen gesendet wurde.

11. Vorrichtung zum Erlangen von Luftqualität für ein Haushaltsgerät, umfassend einen Lüfter und eine Luftqualitätserfassungsvorrichtung, wobei die Vorrichtung zum Erlangen von Luftqualität umfasst:
einen Prozessor; und
einen Speicher zum Speichern von durch den Prozessor ausführbaren Anweisungen;
wobei der Prozessor konfiguriert ist, um durchzuführen:
Steuern bzw. Regeln des Lüfters, sich zu drehen;
Erfassen von Luftqualität durch die Luftqualitätserfassungsvorrichtung;
Erzeugen von Luftqualitätsinformationen gemäß dem Erfassungsergebnis der Luftqualitätserfassungsvorrichtung,
**dadurch gekennzeichnet, dass** der Prozessor weiter konfiguriert ist, durchzuführen:
Erfassen, ob eine Luftströmungsgeschwindigkeit in einer gegenwärtigen Umgebung eine vorbestimmte Geschwindigkeit erreicht;
Steuern bzw. Regeln des Lüfters, sich zu drehen, wenn die Luftströmungsgeschwindigkeit die vorbestimmte Geschwindigkeit nicht erreicht;
Erfassen, ob eine Drehzeit des Lüfters eine vorbestimmte Dauer erreicht;
Erfassen der Luftqualität durch die Luftqualitätserfassungsvorrichtung, wenn die Drehzeit die vorbestimmte Dauer erreicht.

12. Computerprogramm, das, wenn es auf einem Prozessor (718) ausgeführt wird, das Verfahren nach einem der Ansprüche 1 bis 5 durchführt.

## Revendications

1. Procédé d'acquisition de qualité d'air appliqué dans un appareil électroménager comprenant un ventilateur et un dispositif de détection de qualité d'air, et comprenant :
la commande (201) de rotation du ventilateur ;
la détection (202) de qualité d'air par le dispositif de détection de qualité d'air ; et
la génération (203) d'informations de qualité d'air selon le résultat de détection du dispositif de détection de qualité d'air,
**caractérisé en ce que** la commande de rotation du ventilateur comprend :
le fait de détecter si une vitesse de flux d'air dans un environnement actuel atteint une vitesse prédéterminée ; et
la commande de rotation du ventilateur si la vitesse de flux d'air n'atteint pas la vitesse prédéterminée,
et **en ce que** la détection d'air par le dispositif de détection de qualité d'air comprend :
le fait de détecter si un temps de rotation du ventilateur atteint une durée prédéterminée ; et
la détection de la qualité d'air par le dispositif de détection de qualité d'air si le temps de rotation atteint la durée prédéterminée.

2. Procédé selon la revendication 1, **caractérisé en ce que** le procédé comprend en outre :
la réception (301) d'un signal de déclenchement envoyé par un terminal mobile pour déclencher la détection de la qualité d'air ; et
la réalisation (302) d'une opération de commande de rotation du ventilateur après réception du signal de déclenchement.

3. Procédé selon la revendication 2, **caractérisé en ce que**, le procédé comprend en outre :
l'envoi direct des informations de qualité d'air au terminal mobile qui est utilisé pour afficher les informations de qualité d'air ;
et/ou,
l'envoi des informations de qualité d'air à un serveur qui est utilisé pour acheminer les informations de qualité d'air au terminal mobile pour l'affichage des informations de qualité d'air ;
et/ou,
l'envoi des informations de qualité d'air à un dispositif de passerelle domestique qui est utilisé pour acheminer les informations de qualité d'air au terminal mobile pour l'affichage des informations de qualité d'air.

4. Procédé selon la revendication 1, **caractérisé en ce que**, le procédé comprend en outre :
le fait de détecter (401) si un temps prédéterminé est atteint, le temps prédéterminé étant à un intervalle prédéterminé, ou, le temps prédéterminé étant un temps de détection fixé par un utilisateur ; et
la réalisation (402) d'une opération de commande de rotation du ventilateur si le temps prédéterminé est atteint.

5. Procédé selon la revendication 4, **caractérisé en ce que** le procédé comprend en outre :
l'envoi des informations de qualité d'air à un serveur, qui est utilisé pour acheminer toutes les informations de qualité d'air reçues avant ou les informations de qualité d'air reçues la dernière fois à un terminal mobile après réception d'un signal de déclenchement envoyé par le terminal mobile pour l'affichage des informations de qualité d'air reçues ;
et/ou,
l'envoi des informations de qualité d'air à un dispositif de passerelle domestique qui est utilisé pour acheminer toutes les informations de qualité d'air reçues avant ou les informations de qualité d'air reçues la dernière fois à un terminal mobile après réception d'un signal de déclenchement envoyé par le terminal mobile pour l'affichage des informations de qualité d'air reçues.

6. Appareil d'acquisition de qualité d'air appliqué dans un appareil électroménager comprenant un ventilateur et un dispositif de détection de qualité d'air, l'appareil comprenant :
un module de commande de rotation (510) configuré pour commander la rotation du ventilateur ;
un module de détection de qualité d'air (520) configuré pour détecter la qualité d'air par le dispositif de détection de qualité d'air ; et
un module d'acquisition d'informations (530) configuré pour générer des informations de qualité d'air selon le résultat de détection du dispositif de détection de qualité d'air,
**caractérisé en ce que** le module de commande de rotation comprend :
un sous-module de détection de vitesse (611) configuré pour détecter si une vitesse de flux d'air dans un environnement actuel atteint une vitesse prédéterminée ; et
un sous-module de commande de rotation (612) configuré pour commander la rotation du ventilateur lorsque le sous-module de détection de vitesse (611) détecte que la vitesse de flux d'air n'atteint pas la vitesse prédéterminée,
et **en ce que** le module de détection de qualité d'air comprend :
un sous-module de détection de temps (621) configuré pour détecter si un temps de rotation du ventilateur atteint une durée prédéterminée ; et
un sous-module de détection de qualité d'air (622) configuré pour détecter la qualité d'air par le dispositif de détection de qualité d'air lorsque le résultat de détection du sous-module de détection de temps (621) est que le temps de rotation atteint la durée prédéterminée.

7. Appareil selon la revendication 6, **caractérisé en ce que** l'appareil comprend en outre :
un module de réception de signal (640) configuré pour recevoir un signal de déclenchement envoyé par un terminal mobile pour déclencher la détection de la qualité d'air ; et
le module de commande de rotation (510) est configuré pour réaliser une opération de commande de rotation du ventilateur après que le module de réception de signal reçoit le signal de déclenchement.

8. Appareil selon la revendication 7, **caractérisé en ce que** l'appareil comprend en outre :
un premier module d'envoi (650) configuré pour envoyer directement des informations de qualité d'air au terminal mobile qui est utilisé pour afficher les informations de qualité d'air ;
et/ou
un deuxième module d'envoi (660) configuré pour envoyer des informations de qualité d'air à un serveur qui est utilisé pour acheminer les informations de qualité d'air au terminal mobile pour l'affichage des informations de qualité d'air ;
et/ou
un troisième module d'envoi (670) configuré pour envoyer des informations de qualité d'air à un dispositif de passerelle domestique qui est utilisé pour acheminer les informations de qualité d'air au terminal mobile qui est utilisé pour afficher les informations de qualité d'air.

9. Appareil selon la revendication 6, **caractérisé en ce que** l'appareil comprend en outre :
un module de détection de temps (680) configuré pour détecter si un temps prédéterminé est atteint, le temps prédéterminé étant un temps déterminé à un intervalle prédéterminé, ou, le temps prédéterminé étant un temps de détection fixé par un utilisateur ; et
le module de commande de rotation (610) configuré pour réaliser une opération de commande de rotation du ventilateur lorsque le module de détection de temps détecte que le temps prédéterminé est atteint.

10. Appareil selon les revendications 8 et 9, **caractérisé en ce que**, l'appareil comprend en outre :
un quatrième module d'envoi (690) configuré pour envoyer les informations de qualité d'air à un serveur qui est utilisé pour acheminer toutes les informations de qualité d'air reçues avant ou les informations de qualité d'air reçues la dernière fois à un terminal mobile après réception d'un signal de déclenchement envoyé par le terminal mobile pour l'affichage des informations de qualité d'air reçues ;
et/ou,
un cinquième module d'envoi (691) configuré pour envoyer des informations de qualité d'air à un dispositif de passerelle domestique qui est utilisé pour acheminer toutes les informations de qualité d'air reçues avant ou les informations de qualité d'air reçues la dernière fois à un terminal mobile après réception d'un signal de déclenchement envoyé par le terminal mobile pour l'affichage des informations de qualité d'air reçues.

11. Dispositif d'acquisition de qualité d'air pour un appareil électroménager comprenant un ventilateur et un dispositif de détection de qualité d'air, le dispositif d'acquisition de qualité d'air comprenant :
un processeur ; et
une mémoire pour stocker des instructions exécutables par le processeur ;
dans lequel le processeur est configuré pour réaliser :
la commande de rotation du ventilateur ;
la détection de qualité d'air par le dispositif de détection de qualité d'air ;
la génération d'informations de qualité d'air selon le résultat de détection du dispositif de détection de qualité d'air,
**caractérisé en ce que** le processeur est en outre configuré pour réaliser :
le fait de détecter si une vitesse de flux d'air dans un environnement actuel atteint une vitesse prédéterminée ;
la commande de rotation du ventilateur si la vitesse de flux d'air n'atteint pas la vitesse prédéterminée,
le fait de détecter si un temps de rotation du ventilateur atteint une durée prédéterminée ;
la détection de la qualité d'air par le dispositif de détection de qualité d'air si le temps de rotation atteint la durée prédéterminée.

12. Programme d'ordinateur, qui lorsqu'il est exécuté par un processeur (718), réalise le procédé selon l'une quelconque des revendications 1 à 5.
